# EUROPEAN PATENT APPLICATION

(11) **EP 4 260 800 A1**
(43) Date of publication of application: **18.10.2023**
(21) Application number: 22305560.9
(22) Date of filing: 15.04.2022
(51) Int. Cl.: A61B 5/022, A61B 5/0225, A61B 5/00, A61B 5/024, A61B 5/029, A61B 5/08, A61B 5/1455

(54) **WEARABLE BLOOD MONITORING DEVICE AND METHOD**

(71) Applicant: Hinlab, 92200 Neuilly-Sur-Seine (FR)
(72) Inventor: DE BEGON DE LAROUZIERE DE MONTLOSIER, Denys-Michel, 75006 Paris (FR)
(74) Representative: Icosa

(57) **Abstract**

A wearable blood monitoring device is configured to be worn around a limb of a subject and comprises: a cuff configured, in use, to surround the limb of the subject, said cuff comprising an inflatable compartment and a first sensor configured to be in contact with the limb of the subject, the first sensor being configured to acquire a physiological signal from the limb; a second sensor configured to acquire a pressure signal relating to a pressure inside the inflatable compartment; a pumping unit configured to regulate the pressure inside the inflatable compartment, and a control unit configured to inflate the inflatable compartment from a first predefined pressure to a second predefined pressure and acquire a physiological signal from the first sensor while a third predefined pressure is present in the inflatable compartment, said third predefined pressure corresponding to an optimal contact between the first sensor and the limb of the subject and being equal or comprised between the first and second predefined pressures.

## Description

### FIELD OF INVENTION

The present invention relates to a wearable blood monitoring device. The invention further concerns a corresponding method for blood monitoring of a subject. The invention relates to the fields of wearables devices specially adapted for detecting, monitoring or modelling physiological parameters.

### BACKGROUND OF INVENTION

Wearable monitoring devices have gained growing interest as they allow to collect and deliver in real-time various physiological parameters of a subject wearing such devices. The expression "wearable monitoring devices" encompasses various types of devices such as garments, smart watches, or patches, which collect and deliver in real-time physiological parameters of the subject.

One common drawback of these devices is that several sources of noise affect the collected signals, including intrinsic factors *(e.g.,* relating to the device structure, or the type of sensors) and extrinsic factors (*e.g*., environmental temperature, humidity or light). As a result, wearable monitoring devices provide information about fitness and lifestyle, but are not sufficiently accurate to safely take health decisions, and to be used in a hospital setting.

There is a need for improved wearable devices enabling a safe monitoring of the vital signs of the subject. Such devices should be adapted to collect physiological signals from various types of sensors, while ensuring good signal quality and low variability.

The present invention aims to provide wearable devices and methods to acquire physiological signals, and determine vital signs of the subject therefrom, which overcome current limitations. It is also an objective of the present invention to provide wearable devices and methods which allow to collect physiological signal (and calculate physiological parameters therefrom) more accurately and safely.

### SUMMARY

To this end, the present invention is a wearable blood monitoring device of the aforementioned type, configured to be worn around a limb of a subject and comprising:
- a cuff configured, in use, to surround the limb of the subject, said cuff comprising an inflatable compartment and a first sensor configured to be in contact with the limb of the subject, the first sensor being configured to acquire a physiological signal from the limb;
- a second sensor configured to acquire a pressure signal relating to a pressure inside the inflatable compartment;
- a pumping unit configured to regulate the pressure inside the inflatable compartment, and
- a control unit configured to inflate the inflatable compartment from a first predefined pressure to a second predefined pressure and acquire a physiological signal from the first sensor while a third predefined pressure is present in the inflatable compartment, said third predefined pressure being configured to ensure an optimal contact between the first sensor and the limb of the subject and being equal or comprised between the first and second predefined pressures.

The present device allows to implement a dual-sensing and control method which simultaneously monitors the physiological signal and the pressure inside the inflatable compartment. On one hand, the device ensures that the physiological signal is acquired under controlled conditions (which are monitored based on the pressure inside the inflatable compartment), thereby ensuring reproducible measurements. On the other hand, the present device allows to adjust these acquisition conditions as needed *(i.e.,* by adjusting the third predefined pressure, for instance to counteract environmental changes which may have an impact on the quality of the physiological signal), thereby ensuring accurate measurements. Therefore, the device provides a twofold control over the accuracy and the reproducibility of the physiological signal and, accordingly, over any vital sign calculated therefrom.

Moreover, the accuracy is herein guarantee by an *a priori* approach consisting in providing the third predefined pressure which ensures an optimal contact between the first sensor and the limb at the time of the physiological signal acquisition, rather than making assumptions about said contact (which might or might not be fulfilled) and subsequently implementing *a posteriori* corrections. To this end, the cuff advantageously allows to apply a desired and controlled pressure on the limb (based on the third predefined pressure), and also to block the environmental light *(i.e.,* necessary for the use of a PPG sensor). Moreover, the cuff advantageously allows to dynamically apply a wide range of pressures. Accordingly, the third predefined pressure may be selected from a wide range of pressures depending on the subject's characteristics, environmental conditions or other considerations. Furthermore, the control unit allows to control and eventually modify the pressure on the limb as needed, by modifying the third predefined pressure.

According to other advantageous aspects of the invention, the device comprises one or more of the features described in the following embodiment, taken alone or in any possible combination.

In one embodiment, the third and second predefined pressure have an equal pressure value, and the control unit is further configured to maintain said pressure value in the inflatable compartment for a predefined period of time while the physiological signal is acquired from the first sensor. This advantageously allows to acquire the physiological signal while the pressure exerted on the limb is constant, thereby ensuring repeatable and reproducible measurements.

In one embodiment, the third predefined pressure is inferior to the second predefined pressure, and the control unit is further configured to acquire the physiological signal from the first sensor in a time window during which the pressure in the inflatable compartment is equal to the third predefined pressure, during inflation and/or deflation of the inflatable compartment. This embodiment advantageously allows to combine the physiological signal acquisition with an occlusive measurement of the blood pressure of the subject wearing the device.

In one embodiment, the device further comprises a processing unit configured to:
- receive the acquired physiological signal and a value of the pressure inside the inflatable compartment;
- calculate a quality metric of the acquired physiological signal;
- whenever the quality metric of the acquired physiological signal exceeds a predefined quality threshold, define an optimal pressure having the received value of the pressure inside the inflatable compartment, and send instructions to the control unit to set as the third predefined pressure the value of said optimal pressure;
- else, send instructions to the control unit to inflate the inflatable compartment at a newly defined pressure superior to the received value of the pressure, and acquire the physiological signal from the first sensor while the pressure inside the inflatable compartment equals said newly defined pressure.

This embodiment allows to provide a step-wise inflation wherein, at each step, the device tests if the applied pressure is suitable to be used as third predefined pressure, based on the quality metric. If that is not the case, a new, different pressure is tested. Therefore, this embodiment advantageously allows to adapt the third predefined pressure as needed.

In one embodiment, the processing unit is configured to receive the physiological signal acquired from the first sensor and to compute at least one metric of the physiological signal, said at least one metric being chosen among one of the following: perfusion index, signal-to-noise ratio, motion, skewness, kurtosis, entropy, zero crossing rate, systolic wave detector, relative power, or a combination thereof. These quality metrics advantageously provide information about the quality of the physiological signal. This information may be used to adjust the third predefined pressure if needed.

In one embodiment, the first sensor is an optical sensor. In this case, the processing unit is advantageously configured to compute a physiological parameter being chosen among one of the following: oxygen saturation, heart rate, blood pressure, respiratory rate, blood pressure trend, heart rate variability, or cardiac output. The device advantageously allows to improve the accuracy of these parameters by reducing, among others, motion artifacts and ambient light.

In one embodiment, the control unit is configured to:
- inflate at a predefined pressure the inflatable compartment and deflate it from the first predefined pressure to the second predefined pressure,
- acquire a pressure inside the inflatable compartment using the second sensor during a time window during inflation or deflation.

This embodiment advantageously allows to acquire the physiological signal while the pressure exerted on the limb is constant and to combine the physiological signal acquisition with an occlusive measurement of the blood pressure of the subject.

In one embodiment, the device further comprises at least one third sensor. In this case, the control unit is configured to acquire a physiological signal from said third sensor, said third sensor being chosen among the following: accelerometer, gyroscope, at least one electrode, an ultrasound sensor, a tomographic sensor, an acoustic sensor, a magnetometer, a humidity sensor, a temperature sensor, a heat-flux sensor. This embodiment advantageously allows to monitor several physiological parameters of the subject. For example, physiological parameters relating to blood pressure and physiological parameters relating to body temperature.

The present invention further relates to a method for blood monitoring of a subject, the method being implemented by a wearable blood monitoring device according to any one of the embodiments described hereinabove, and comprising the following steps:
- inflating the inflatable compartment;
- acquiring the pressure signal relating to a pressure inside the inflatable compartment from the second sensor;
- when the pressure inside the inflatable compartment equals the third predefined pressure, acquiring the physiological signal from the first sensor.

In one embodiment, during the step of inflating, the inflatable compartment is inflated at the third predefined pressure, the method further comprises a step of maintaining said third predefined pressure in the inflatable compartment for a predefined period of time, and the step of acquiring the physiological signal from the first sensor is performed during said predefined period of time.

In this embodiment, the physiological signal is acquired while the pressure exerted on the limb is constant.

In one embodiment, the method comprises a step of inflating the inflatable compartment from the first to the second predefined pressure and/or deflating the inflatable compartment from the second to the first predefined pressure, wherein the third predefined pressure is inferior to the second predefined pressure, and the step of acquiring the physiological signal from the first sensor is performed in a time window during which the pressure in the inflatable compartment is equal to the third predefined pressure, during said step of inflating and/or deflating. This embodiment advantageously allows to combine the physiological signal acquisition with an occlusive blood pressure measurement.

In one embodiment, the method further comprises:
- receiving the acquired physiological signal and a value of the pressure inside the inflatable compartment during the acquiring;
- evaluating a quality metric of the acquired physiological signal;
- whenever the quality metric of the acquired physiological signal exceeds a predefined quality threshold, defining an optimal pressure having the value of the pressure inside the inflatable compartment during the acquiring, and setting, as the third predefined pressure, the value of said optimal pressure;
- else, inflating the inflatable compartment at a newly defined pressure, superior to the received value of pressure, and acquiring the physiological signal from the first sensor while the pressure inside the inflatable compartment equals said newly defined pressure.

This embodiment advantageously allows to modify the third predefined pressure based on variation in the physiological signal quality. Accordingly, the specificity of the third predefined pressure for the subject is ensured over time.

In one embodiment, the predefined quality threshold is selected among one of the following: perfusion index, signal-to-noise ratio, motion, skewness, kurtosis, entropy, zero crossing rate, systolic wave detectors, relative power, or a combination thereof.

In one embodiment, the steps of the method are periodically repeated or user-initiated. By repeating the steps periodically, it is possible to provide a longitudinal monitoring of the subject over time. Moreover, this embodiment enables the healthcare providers to regularly receive information about the health of the subject, thereby ensuring rapid responses. By performing the steps after a user request (user-initiated steps), it is possible to perform a spot check monitoring of the subject.

In the present invention the user initiating the device/method may be the subject wearing the device or a healthcare provider assisting the subject.

### DEFINITIONS

In the present invention, the following terms have the following meanings.

*"About"* is used herein to mean approximately, roughly, around, or in the region of. When the term *"about"* is used in conjunction with a numerical range, it modifies that range by extending the boundaries above and below the numerical values set forth. When the term *"about"* is used preceding a figure means plus or less 10% of the value of said figure.

The terms *"adapted"* and *"configured* are used in the present disclosure as broadly encompassing initial configuration, later adaptation or complementation of the present device, or any combination thereof alike, whether effected through material or software means (including firmware).

The term *"between"* or *"between about"* referring to a range of values means including both endpoints of the stated range of values.

The term *"physiological signal"* refers to a signal representative of a physiological process(es) taking place in the body of the subject and that is collected via sensor(s) thanks to different detection and acquisition techniques known by the skilled person. These signals representative of a physiological process may be transmitted by the sensor for further processing. In the context of the present invention, non-limitative examples of physiological signals are: acoustic signals *(e.g.,* representative, *i.e.,* produced, by the flow of blood in the circulatory system, or by breathing cycles); optical signals (*e.g*., produced by light transmission and reflectance from a tissue).

The terms *"processor"* (or *"processing unit"*) should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

The term *"quality metrics"* refers to a statistical parameter that is calculated from a signal measured from a sensor and that indicates how good and reliable said signal is, *i.e.,* how small is the unwanted component of the signal *(e.g.,* background noise, crosstalk, movement artifacts, etc.), when compared to the desired component, which is specific to the phenomenon being measured.

### BRIEF DESCRIPTION OF THE DRAWINGS

The invention will be better understood with the attached figures, in which:
- **Figure 1** is a perspective view of a wearable device 1 according to the invention;
- **Figure 2** is a schematic representation of a first embodiment of the wearable device 1 for blood monitoring according to the present invention;
- **Figure 3** is a flowchart representing a first embodiment of a method for blood monitoring of a subject according to the invention;
- **Figure 4** is a graph showing a first example of pressure inside the inflatable compartment of the wearable device 1 according to the invention as a function of time;
- **Figure 5** is a graph showing a second example of pressure inside the inflatable compartment of the wearable device 1 according to the invention as a function of time;
- **Figure 6** is a flowchart representing a second embodiment of the method for blood monitoring of the subject according to the invention;
- **Figure 7** is a flowchart representing a third embodiment of the method for blood monitoring of the subject according to the invention;
- **Figure 8** is a graph showing a third example of pressure inside the inflatable compartment of the wearable device 1 according to the invention as a function of time;
- **Figure 9** is a graph showing a fourth example of pressure inside the inflatable compartment of the wearable device 1 according to the invention as a function of time;
- **Figure 10** is a flowchart representing a fourth embodiment of the method for blood monitoring of the subject according to the present invention;
- **Figure 11** is a combination of graphs. **Figure 11A** shows the physiological signal obtained from the first sensor 12 while the inflatable compartment 101 is deflated, and **figure 11B** shows the perfusion index PI calculated from the signal of **figure 11A****;**
- **Figure 12** is a combination of graphs. **Figure 12A** shows the physiological signal from the first sensor 12 while the third predefined pressure is present in the inflatable compartment 101; and **figure 12B** shows the perfusion index PI calculated from the signal of **figure 12A****.**

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, *i.e.,* any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

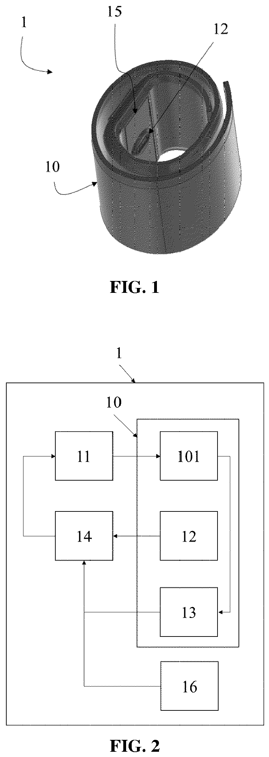

The present disclosure will be described in reference to a particular functional embodiment of a wearable blood monitoring device, as illustrated on **figure 1****.**

As shown in **figure 1****,** the device 1 comprises a cuff 10 configured, in use, to surround the limb of the subject. The cuff 10 may be removably secured to one limb of the subject through the use of fastening means such as a hook-and-loop attachment, a buckle, a magnet. However, the presently disclosed subject-matter is not limited to any particular way in which the cuff 10 is secured to the limb. Preferably, the cuff 10 is configured to be worn around an upper limb of the subject. Indeed, the upper limbs have a large vascular network, which allows the device 1 to acquire physiological signals caused, or influenced, by blood flow *(e.g.,* relating to blood-oxygen levels, pulse transit time, blood pressure, blood volume, heart rate and the like). Moreover, when compared to other body appendages, the upper limbs have less cartilage and bone which are a source of noise and may affect the acquisition of physiological signals. Advantageously, the upper limbs are also less sensitive to motion artifacts.

As schematically shown in **figure 2****,** the cuff 10 comprises an inflatable compartment 101. This inflatable compartment 101 may occupy the entire cuff 10 (*i.e.,* the whole cuff is inflatable) or just a portion of the cuff 10. The inflatable compartment 101 is preferably made of plastic fibers, TPU, PVC, or a combination thereof and/or of coated fabrics such as Nylon^{™}. These materials are resistant to pressure thereby allowing to inflate the inflatable compartment 101 without risk of tearing. Moreover, they are well-tolerated by the skin.

The device 1 further comprises a pumping unit 11 configured to regulate the pressure inside the inflatable compartment 101. For example, the pumping unit 11 may be a rolling air pump, a piezo electric pump, a resonant gas pump, a diaphragm/membrane gas pump, and the like. The pumping unit 11 may further comprise one or more active or passive valves (*e.g*., blowers). The valve may be normally-open or normally-closed. Preferably, the pumping unit 11 comprises a normally-closed valve. This type of valve allows to provide a constant pressure inside the inflatable compartment 101 while

The device 1 may further comprises a first sensor 12 configured to acquire a physiological signal. Said first sensor 12 is configured to be in contact with the limb of the subject when the device 1 is in use. For instance, it may be arranged at a location of the cuff 10 in proximity of the inflatable compartment 101.

For example, the first sensor 12 may be located in a non-inflatable portion of the cuff 10, notably, in a dedicated casing 15 designed to house sensors or other electronic parts. In other examples, the first sensor 12 may be laid onto the inflatable compartment 101, for instance integrated in a printed circuit layer onto the inflatable compartment 101. Said first sensor 12 is preferably an optical sensor such as a photoplethysmographic (PPG) sensor.

The device 1 further comprises a second sensor 13 configured to acquire a pressure signal relating to a pressure inside the inflatable compartment 101. The second sensor 13 may be laid onto the inflatable compartment 101. In some cases, the second sensor 13 is wired or wirelessly connected to a control unit 14 (which will be described later). In other examples, the second sensor 13 is located in a casing 15. Preferably, the second sensor 13 and the control unit 14 are located in the same casing 15. This embodiment advantageously allows to avoid risks which may be associated with the presence of electrical connections between the second sensor 13 and the control unit 14.

Advantageously, by positioning the second sensor 13 and the control unit 14 (and, optionally, the other sensors 12, 16 or electronic parts which may be comprised in the device 1) inside said casing 15, it is possible to reduce the number of manufacturing operations carried out on the inflatable compartment 101. This advantageously allows to provide a simpler and faster manufacturing process. Moreover, the device 1 is more compact.

Optionally, the device 1 may comprise at least one third sensor 16 in addition to the first and second sensors 12, 13. Said at least one third sensor 16 is preferably selected among: accelerometer, gyroscope, at least one electrode, ultrasound sensor, tomographic sensor, acoustic sensor, magnetometer, humidity sensor, temperature sensor and/or heat-flux sensor. The at least one third sensor 16 may be integrated in the cuff 10, or it may be external to the cuff *(e.g.,* attached to a different body portion).

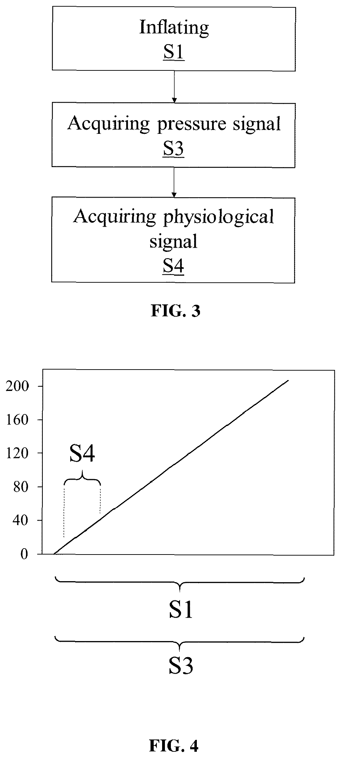

The device 1 further comprises a control unit 14. The control unit 14 comprises circuits for controlling the pumping unit 11 *(i.e.,* driving a motor of the pump), so as to inflate the inflatable compartment 101 at the desired pressures. **Figure 3** illustrates the main steps implemented by said control unit 14. More precisely, the control unit 14 is configured to inflate the inflatable compartment from a first predefined pressure to a second predefined pressure.

The first predefined pressure may correspond to about 0 mm Hg. The second predefined pressure is preferably higher than 50 mm Hg. In some cases, the second predefined pressure may be higher. For instance, it may be comprised between 180 mm Hg and 300 mm Hg. This embodiment advantageously allows to occlude blood vessels of the limb if needed, for instance in order to perform an occlusive blood pressure measurement.

The control unit 14 is further configured to acquire S3 the pressure signal from the second sensor 13. Whenever one or more third sensors 16 are present, the control unit 14 may further be configured to acquire signals from these sensors 16. Moreover, the control unit 14 is configured to acquire S4 a physiological signal from the first sensor 12 while a third predefined pressure is present in the inflatable compartment 101. The third predefined pressure is preferably comprised between 5 mm Hg and 80 mm Hg.

In some examples, the third predefined pressure is comprised between 10 mm Hg and 40 mm Hg, preferably between about 20 mm Hg and 30 mm Hg. Advantageously, these pressures ensure, in most subjects, optimal contact between the first sensor 12 and the limb and comfort of the subject.

Of note, the instructions configured to control the pumping unit 11 *(i.e.,* inflating S1) and acquisition of (S3, S4) signals using any one of the sensors (12, 13, 16) may be provided by a single control unit 14 (**figure 2**). This allows to obtain an advantageously compact device 1. However, in some examples, the device 1 may comprise a control unit 14 and a separated processing unit respectively configured to implement these functions. This allows to the functions in parallel, thereby increasing computational efficiency.

In this case, the control unit 14 (for instance, a motor driver) may be configured to receive as input an initial voltage supplied by the processing unit (for instance, a microcontroller). The device 1 may further comprise a voltage regulator configured to receive the initial voltage supplied by the processing unit and to provide a lower voltage to the control unit 14. Alternatively, or in addition, the device 1 may comprise a booster configured to receive said initial voltage and to provide a higher voltage to the control unit 14. The control unit 14 may further be configured to be powered by a power supply unit (not shown). The power supply unit may be a battery embedded in the wearable device 1, or an external power supply unit.

A common problem observed during signal acquisition from sensors located in a wearable device is that these devices do not optimally conform to the shape and curvatures of the body. Another problem observed during signal acquisition from sensors located in a wearable device is that the sensor detaches from the skin, due to transpiration, skin condition, environmental factors, and to the material of the wearable device. For instance, adhesive patches lose their adhesive properties, and tight garments become loose over time. These problems result in low signal quality due to suboptimal contact; motion artifacts due to sensor displacement from its original position; and high noise levels due to the incidence of ambient light on the body tissues facing the sensor. Moreover, in worse cases, the signal variations may erroneously be interpreted as real variation in a physiological parameter of the subject.

To alleviate these issues, the control unit 14 of the wearable device 1 is configured to acquire the physiological signal while the pressure inside the inflatable compartment 101 pressure is equal to a third predetermined pressure. Advantageously, the third predefined pressure is high enough to ensure an optimal contact between the first sensor 12 and the limb and to block the ambient light, but small enough to ensure the comfort of the subject wearing the present device. Moreover, the pressure exerted by the inflatable compartment 101 on the limb, due to the presence of said third predetermined pressure, also blocks ambient light. Said third predefined pressure may be greater than the first predefined pressure and smaller than the second predefined pressures, or equal to the second predefined pressure.

**Figure 4** is a graph showing an exemplary relationship between the pressure inside the inflatable compartment 101 (in mm Hg) over time. Said relationship represents an inflation profile of the inflatable compartment 101.

The control unit 14 may be configured to provide a fixed inflation rate, or to adjust the inflation rate based on the third predefined pressure and a desired inflation time of the inflatable compartment 101 (for instance, between 30 seconds and 180 seconds for adults, or 90 seconds for neonates, see ISO 80601-2-61). In addition, the control unit 14 may be configured to determine the inflation rate based on parameters such as the quality of the signals collected from the sensors (12, 13, 16).

In this example, the third predefined pressure is smaller than the second predefined pressure. More precisely, the control unit 14 is configured to inflate the inflatable compartment 101 from 0 mm Hg (first predefined pressure) to 120 mm Hg (second predefined pressure) and to acquire the physiological signal while the pressure inside the inflatable compartment 101 (third predefined pressure) is between 50 mm Hg and 80mm Hg.

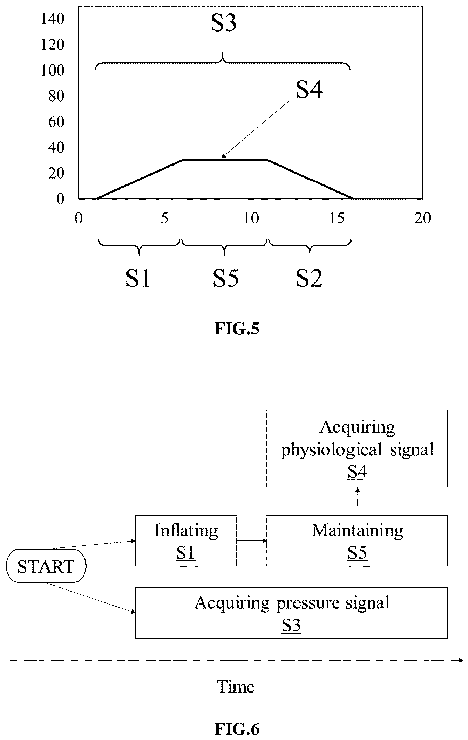

**Figure 5** illustrates another inflation profile that may be obtained by the device 1 in which the third predefined pressure is equal to the second predefined pressure (flat portion of the inflation profile). In this example, the control unit 14 is further configured to maintain the pressure stable for a predefine time and then deflate the inflatable compartment 101 after acquisition of the physiological signal.

The control unit 14 may be configured to acquire the physiological signal at a constant pressure *(i.e.,* at the same third predetermined pressure). This embodiment is also illustrated by the example of **figure 5****.** This feature advantageously allows to provide the stable conditions (*e.g*., constant level of ambient light obstruction, constant pressure exerted on the limb) during the time of the acquisition, thereby ensuring repeatable and reproducible measurements.

**Figure 6** schematically illustrates the main steps implemented by the control unit 14 to provide the acquisition of the physiological signal at constant pressure. In this case, the control unit 14 may be configured to:
- inflate the inflatable compartment 101;
- during said inflation, acquire the pressure signal from the second sensor 13;
- when said pressure signal indicates that the pressure inside the inflatable compartment 101 is equal to the third predefined pressure, maintain said third predefined pressure in the inflatable compartment 101 for a predefined period of time;
- acquire the physiological signal from the first sensor 12 during said predefined period of time.

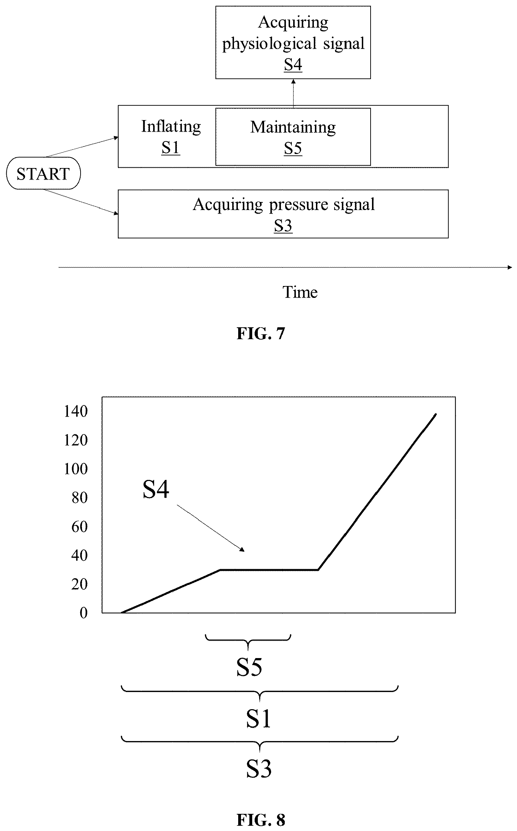

Preferably, the control unit 14 is configured to maintain the third predetermined pressure for less than 15 minutes. More preferably, the control unit 14 is configured to maintain the third predetermined pressure for a time comprised between 20 seconds and 2 minutes. The control unit 14 may be configured to deflate (**figure 5**) or to further inflate (**figure 7**) the inflatable compartment 101 at the end of the predefined period of time.

**Figure 8** shows an exemplary inflation profile provided by the control unit 14 of **figure 7****.**

Of note, providing a control unit 14 configured to further inflate the inflatable compartment after the acquisition of the physiological signal (as illustrated in **figures 4** and **8**), advantageously allows to combine the physiological signal acquisition with an occlusive measurement of the blood pressure. In this case, as aforementioned, the second predefined pressure should be high enough to temporally occlude blood flow *(e.g.,* to occlude the brachial artery for a device 1 configured to be worn around the upper limb). Another advantage of this embodiment is the possibility to calculate several vital signs of the subject *(i.e.,* a physiological parameter at the third predefined pressure, and a blood pressure at a greater, occlusive, pressure) in during a single inflation profile, rather than calculate them in different runs. This allows to reduce inter-experimental variability. The acquisition of the physiological signal after or during an inflation (**figures 4****,** **5** and **8**), advantageously allows to avoid signal artifacts which may occur during or after a deflation, such as small increases in the perfusion index resulting from the release of blocked blood.

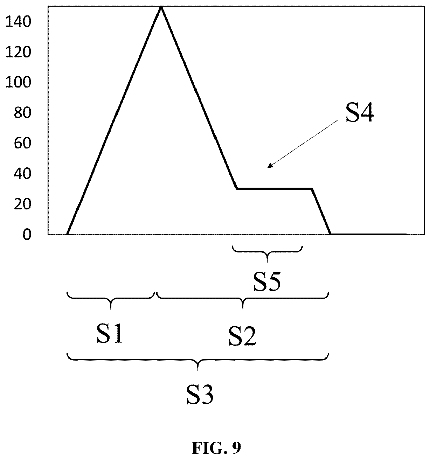

However, in some cases, it may be desirable to acquire the physiological signal during a deflation. One example of this embodiment is shown in **figure 9****.** More precisely, **figure 9** illustrates an inflation profile comprising an inflation from 0 mm Hg to 140 mm Hg, followed by a deflation back to 0 mm Hg, and wherein the control unit 14 is configured to maintain the third predefined pressure (during a maintaining step S5) and acquire the physiological signal during the deflation.

It should be understood that the inflation profiles provided by the control unit 14 are not limited to the examples described hereinabove. For instance, the control unit 14 may be configured to provide an inflation profile comprising a step-wise inflation or a step-wise deflation. In this case, the control unit 14 may be configured to acquire the signal from the first sensor 12 at each step, *i.e.,* at each flat portion of the inflation profile.

Preferably, the control unit 14 is configured to inflate or deflate the inflatable compartment linearly. In other words, the inflation profile is made of straight lines. To this end, the control unit 14 preferably comprises a Proportional-Integral-Derivative (PID) controller. The PID controller *(e.g.,* PID velocity or PID position) is configured to receive, as input, the pressure signal from the second sensor 13, and to determine whether a slope of the pressure profile corresponds to a desired slope. In other words, the PID controller allows to ensure the linearity of the inflation and deflation. Therefore, this embodiment advantageously allows to improve the quality of the physiological signal and its consequent processing (e.g., extraction of an oscillometric pulse envelope from the inflation profile).

The device 1 may further comprise a memory. The memory may be configured to store one or more third predefined pressures. In this case, the control unit 14 is configured to retrieve the third predefined pressure(s) from the memory.

The device 1 may interact with a user interface, via which information can be entered and retrieved by a user. The user interface includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system. Optionally, the third predefined pressure may be a value inputted by a user via said user interface. Advantageously, the user interface also permits to receive inputs or external commands (*e.g.,* a request of launching or interrupting a signal acquisition or an inflation) from a healthcare provider if needed, and/or to output one or more vital signs of the subject calculated by the control unit 14.

The control unit 14 may further be configured to select and/or update the third predefined pressure based on one or more quality metrics. To this end, the control unit 14 may be configured to receive the acquired physiological signal and the value of the pressure inside the inflatable compartment 101.

The acquired physiological signal may be used to calculate at least one quality metric while the corresponding value of the pressure inside the inflatable compartment 101 may be used to define an optimal pressure when the quality metrics meet one or more requirements. The calculation of the quality metrics and the definition of the optimal pressure will now be described in further details.

The at least one quality metric may be calculated based on various features of the acquired physiological signal, in time domain or frequency domain (*e.g.,* amplitude, steepness, phase features, and the like). For instance, the at least one quality metric may be calculated based on the amplitude of the acquired physiological signal *(e.g.,* light intensity for a physiological signal acquired from an optical sensor).

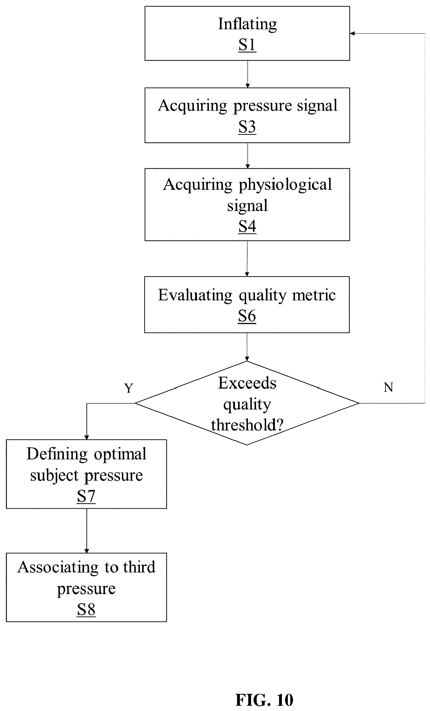

**Figure 10** schematically illustrates steps implemented by the control unit 14 in this case. In this example, the control unit 14 is configured to define an optimal pressure based on the received value of the pressure inside the inflatable compartment 101. More precisely, whenever the quality metric of the acquired physiological signal exceeds a predefined quality threshold, the control unit 14 sets the corresponding value of pressure inside the inflatable compartment 101 as the optimal pressure. Furthermore, the control unit 14 is configured to set as the third predefined pressure the value of said optimal pressure.

The control unit 14 may also be configured to inflate the inflatable compartment 101 at a newly defined pressure (superior to the received value of the pressure) if the quality metric does not exceed the predefined quality threshold. In this case, the control unit 14 is configured to acquire the physiological signal using the first sensor 12 while the pressure inside the inflatable compartment equals said newly defined pressure. This embodiment advantageously allows to provide an auto-adjustable device capable of: verifying whether the control unit 14 inflates the inflatable compartment 101 to a pressure suitable to be used as third predefined pressure, and replacing a previous third predefined pressure with a newly defined pressure if needed. More precisely, the third predefined pressure is replaced by the newly defined pressure in response to the identification of a suboptimal quality of the physiological signal *(e.g.,* due to a movement of the subject, or environmental conditions which are identified thanks to the quality metrics calculation), to ensure a better quality for the next acquired physiological signal.

Preferably, the control unit 14 is configured to calculate at least one quality metric for the acquired physiological signal. Said quality metrics is selected among one of the following: perfusion index, signal-to-noise ratio (SNR), motion, skewness, kurtosis, entropy, zero crossing rate, systolic wave detector and/or relative power. Other pertinent quality metrics known by the person skilled in the art could be as well implemented. The control unit 14 may be configured to calculate the quality metric as a function and/or combination of quality metrics. Examples of said combination include: a matching of multiple systolic wave detectors, or a combination of perfusion indexes.

Some examples of quality metrics will now be described. As aforementioned, the quality metric may be a perfusion index. In this case, the control unit 14 may be configured to inflate the inflatable compartment at the newly defined pressure if the perfusion index is less than 0,05%.

The quality metrics may be an R-value (also called ratio of ratios), which is calculated as (AC1/ DC1) / (AC2/DC2), wherein AC1 and AC2 are the pulsatile components of the physiological signal each being acquired at a respective wavelength, and DC1 and DC2 are the non-pulsatile components of the physiological signal at the same wavelengths. Calculating the quality metrics based on the R-value advantageously allows to detect a suboptimal signal quality due to motion of the subject. Moreover, it allows to more accurately determine the oxygen saturation of the subject, since this physiological parameter is greatly affected by the R-value.

The quality metrics may be a SNR of the pulsatile component of the physiological signal (for instance, the AC component of a PPG signal). This quality metrics allows to detect a suboptimal signal quality due to lack (or loss) of adherence between the first sensor 12 and the limb or, for an optical first sensor 12, suboptimal signal quality may be due to optical noise.

By *"optical noise"* it is meant a noise occurring on the optical path between the sensor and the limb of the subject, which may be linked, among others, to: presence of air gap between the first sensor 12 and the skin of the subject; thickness of cover glass protecting the sensor (if any); the distance between a light-emitting diode (LED) and the corresponding photodetector; and/or intrinsic characteristics of the LED drivers controlling the current of the LED.

In addition, the quality metrics may be a SNR of the static (non-pulsatile) component of the physiological signal (for instance, the DC component of a PPG signal). The quality metrics may be a SNR of the baseline component of the physiological signal (for instance, the DC component of a PPG signal). The control unit 14 may be configured to periodically calculate the quality metric(s).

As aforementioned, the control unit 14 is further configured to compute a physiological parameter from the physiological signal acquired from the first sensor 12.

The first sensor 12 may be an optical sensor. In this case, the physiological parameter, that may be calculated from the physiological signal, may be selected among one of the following: oxygen saturation, heart rate, blood pressure, respiratory rate, blood pressure trend, heart rate variability, or cardiac output.

In presence of separate control unit 14 and processing unit, the aforementioned computations *(i.e.,* of the physiological parameter and/or of the quality metrics) are performed by the latter. In this case, the processing unit is configured to send instructions to the control unit 14 to set the value of said optimal pressure as the third predefined pressure.

The processing unit is also configured to send instructions to the control unit 14 to inflate the inflatable compartment at a newly defined pressure (superior to the received value of the pressure) if the quality metric does not exceed the predefined quality threshold. Compared to textiles, the device 1 of the invention advantageously allows to apply a wide and controlled range of pressures on the limb of the subject, notably comprised between the first and the second predetermined pressures. The pressure is notably controlled during time to obtain the predefined desired pressure profiles.

Accordingly, the third predetermined pressure may be selected in this range depending on characteristics of the subject (*e.g.,* weight, body mass index, skin condition, blood perfusion, etc.), in order to ensure an optimal contact between the first sensor 12 and the limb of the subject. Moreover, the third predetermined pressure may be modified if needed, in order to ensure that the optimal contact between the first sensor 12 and the limb of the subject is maintained over time. These advantages are not achievable with conventional wearable devices such as adhesives, patches, and compressive garments, because the pressure exerted on the skin by these wearable devices are small. Moreover, these pressures cannot be controlled and modified as needed.

Operation of the device 1 according to the invention will now be described.

During the inflating step S1, the control unit 14 inflates the inflatable compartment 101. During the deflating step S2 the inflatable compartment 101 from the second to the first predefined pressure. During a first acquiring step S3, the control unit 14 acquires the pressure signal from the second sensor 13.

Said inflating step S1 (or deflating step S2) and first acquiring step S3 may occur simultaneously, *i.e.,* the control unit 14 acquires the pressure signal from the second sensor 13 throughout the inflation (or deflation). Then, during a second acquiring step S4, the control unit 14 acquires a physiological signal from the first sensor 12 while a third predefined pressure is present in the inflatable compartment 101.

During an optional maintaining step S5, the control unit 14 maintains the third predefined pressure in the inflatable compartment 101 for a predefined period of time. In this case, the second acquiring step S4 and the maintaining step S5 may occur simultaneously, *i.e.,* the control unit 14 acquires the physiological signal from the first sensor 12 throughout the maintaining step S5.

During an evaluating step S6, the control unit 14 calculates a quality metric of the acquired physiological signal. Then, whenever the quality metric of the acquired physiological signal exceeds a predefined quality threshold, the control unit 14 defines, during a defining step S7, an optimal pressure having the value of the pressure inside the inflatable compartment during the acquiring. In this case, during a setting step S8, the control unit 14 sets, as the third predefined pressure, the value of said optimal pressure.

Otherwise, during another inflating step S1, the control unit 14 inflates the inflatable compartment at the newly defined pressure, superior to the received value of pressure.

Moreover, during another acquiring step S4, the control unit 14 acquires the physiological signal from the first sensor 12 while the pressure inside the inflatable compartment 101 equals said newly defined pressure.

The control unit 14 may periodically repeat the aforementioned steps. These steps may also be user-initiated.

### EXAMPLE

The present invention is further illustrated by the following example.

### Materials and Methods

The experiment employed a group of 29 participants. Participants' ages ranged from 18 to 65 years with 51,7% of participants in the range of 18-30 years, 24,1% in the range of 31-50 years, and 24,1% in the range of 51-65 years. 69% of participants were women, and 31% of them were men. The body mass index (BMI) was in the normal weight range (between 18,5 and 24,9) for 62.1% of participants and in the overweight range (between 25 and 30) for 37,9% of participants. Skin tones were categorized using the Fitzpatrick scale. 65.5% of participants belonged to type I and type II classes, 31% to type III and IV classes, and 3.4% to type V and VI classes.

For this study, the first sensor 12 of the device 1 was a PPG sensor comprising a green LED, a red LED, and an infrared LED. Each participant was demanded to wear the device 1 on the left upper arm, and the signal from the PPG sensor was acquired in a first condition, while the inflatable compartment 101 was deflated, and in a second condition, while the inflatable compartment 101 was inflated and a third predefined pressure was present in the inflatable compartment 101. For this study, a third predefined pressure of 30 mm Hg was selected.

For each of the two conditions, the perfusion Index (PI) was calculated based on the infrared PPG signal. The average PI was further calculated for each condition.

### Results

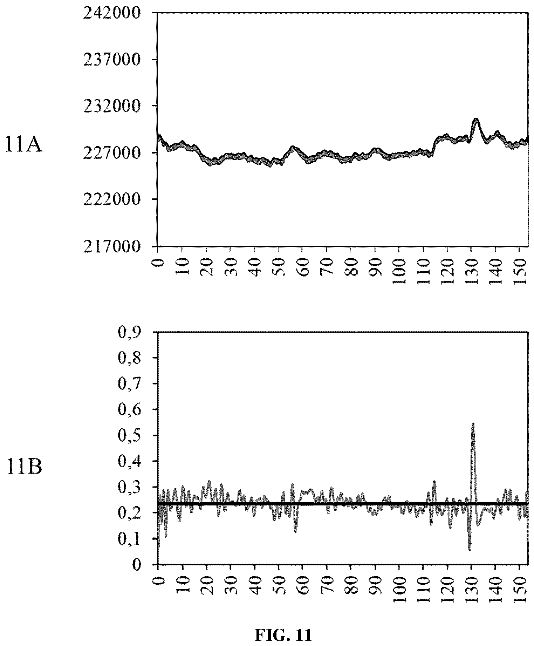

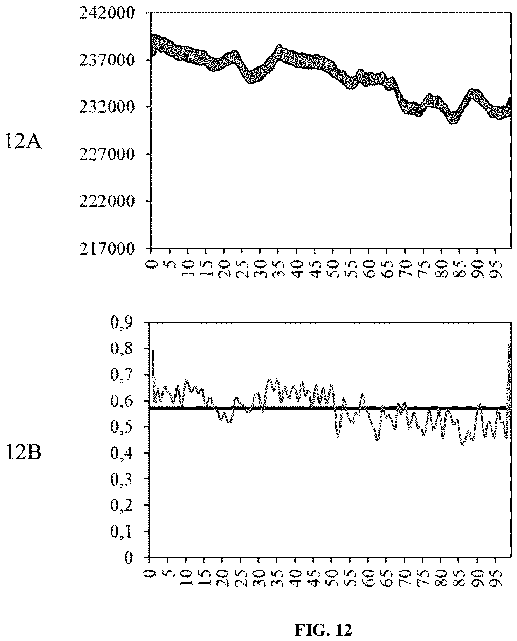

Overall, the average PI of participants was increased by a factor of 1,5 in the second condition, compared to the first condition. The results obtained from one participant of the present study are shown in **figure 11** and **12****.** In this case, the average PI was increased by a factor of 2,4 in the second condition, compared to the first condition. More precisely, **figure 11A** is a graph showing the upper and lower envelopes of the infrared PPG signal (vertical axis, amplitude) as a function of time (horizontal axis, seconds) in the first condition, *i.e.,* while the inflatable compartment 101 is deflated. **Figure 11B** is a graph showing the corresponding PI (grey line, vertical axis, %), *i.e.,* the PI calculated from the signal of **figure 11A** as the normalized ratio of AC and DC signal components. The horizontal black line indicates the average PI.

The graphs of **figure 12** show the infrared PPG signal (**figure 12A**), and the PI (**figure 12B****,** grey line) and average PI (**figure 12B****,** black line) in the second condition, from the same participant of **figure 11****.** The units of the data represented in the graphs of **figure 12** are the same of **figure 11****.** For this participant, the average perfusion index was 0,235 in the first condition and 0,569 in the second condition. These results show that the signal quality is increase in the second condition, *i.e.,* when the third predefined pressure is present in the inflatable compartment 101.

## Claims

1. A wearable blood monitoring device (1) configured to be worn around a limb of a subject, the device (1) comprising:
- a cuff (10) configured, in use, to surround the limb of the subject, said cuff (10) comprising an inflatable compartment (101) and a first sensor (12) configured to be in contact with the limb of the subject, said first sensor (12) being configured to acquire a physiological signal from the limb;
- a second sensor (13) configured to acquire a pressure signal relating to a pressure inside the inflatable compartment (101);
- a pumping unit (11) configured to regulate the pressure inside the inflatable compartment (101), and
- a control unit (14) configured to inflate the inflatable compartment (101) from a first predefined pressure to a second predefined pressure, and acquire a physiological signal from the first sensor (12) while a third predefined pressure is present in the inflatable compartment (101); said third predefined pressure being configured to ensure an optimal contact between the first sensor (12) and the limb of the subject, and being equal or comprised between the first and the second predefined pressures.

2. Device (1) according to claim **1,** wherein the third and second predefined pressures have an equal pressure value, and the control unit (14) is further configured to maintain said pressure value in the inflatable compartment (101) for a predefined period of time while the physiological signal is acquired from the first sensor (12).

3. Device (1) according to claim **1,** wherein the third predefined pressure is inferior to the second predefined pressure, and the control unit (14) is further configured to acquire the physiological signal from the first sensor (12) in a time window during which the pressure in the inflatable compartment (101) is equal to the third predefined pressure, during inflation and/or deflation of the inflatable compartment (101).

4. Device (1) according to claim **2,** further comprising a processing unit configured to:
- receive the acquired physiological signal and a value of the pressure inside the inflatable compartment (101);
- calculate a quality metric of the acquired physiological signal; and
- whenever the quality metric of the acquired physiological signal exceeds a predefined quality threshold, define an optimal pressure having the received value of the pressure inside the inflatable compartment (101), and send instructions to the control unit (14) to set as the third predefined pressure the value of said optimal pressure;
- else, send instructions to the control unit (14) to inflate the inflatable compartment at a newly defined pressure superior to the received value of the pressure, and acquire the physiological signal from the first sensor (12) while the pressure inside the inflatable compartment (101) equals said newly defined pressure.

5. Device (1) according to any one of claims **1** to **4,** wherein the processing unit is further configured to receive the physiological signal acquired from the first sensor (12) and to compute at least one quality metric of the physiological signal, said at least one quality metric being chosen among one of the following: perfusion index, signal-to-noise ratio, motion, skewness, kurtosis, entropy, zero crossing rate, systolic wave detector, relative power, or a combination thereof.

6. Device (1) according to any one of claims **1** to **5,** wherein the first sensor (12) is an optical sensor and the processing unit is further configured to compute a physiological parameter from the physiological signal acquired from the first sensor (12), said physiological parameter being chosen among one of the following: oxygen saturation, heart rate, blood pressure, respiratory rate, blood pressure trend, heart rate variability, or cardiac output.

7. Device (1) according to any one of claims **1** to **6,** wherein the control unit (14) is configured to:
- inflate at a predefined pressure the inflatable compartment (101) and deflate it from the first predefined pressure to the second predefined pressure,
- acquire a pressure inside the inflatable compartment (101) using the second sensor (13) during a time window during inflation or deflation.

8. Device (1) according to any one of claims **1** to **7,** further comprising at least one third sensor (16), and wherein the control unit (14) is further configured to acquire a physiological signal from said third sensor (16); said third sensor being chosen among the following: accelerometer, gyroscope, at least one electrode, an ultrasound sensor, a tomographic sensor, an acoustic sensor, a magnetometer, a humidity sensor, a temperature sensor, a heat-flux sensor.

9. A method for blood monitoring of a subject, the method being implemented by a wearable blood monitoring device (1) according to any one of claims **1** to **8** and comprising the following steps:
- inflating (S1) the inflatable compartment (101);
- acquiring (S3) the pressure signal relating to a pressure inside the inflatable compartment (101) from the second sensor (13);
- when the pressure inside the inflatable compartment (101) equals the third predefined pressure, acquiring (S4) the physiological signal from the first sensor (12).

10. The method according to claim **9,** wherein, during the step of inflating (S1), the inflatable compartment (101) is inflated at the third predefined pressure, the method further comprising a step of maintaining (S5) said third predefined pressure in the inflatable compartment (101) for a predefined period of time, and the step of acquiring (S4) the physiological signal from the first sensor (12) is performed during said predefined period of time.

11. The method according to claim 9, the method comprising a step of inflating (S1) the inflatable compartment (101) from the first to the second predefined pressure and/or deflating (S2) the inflatable compartment (101) from the second to the first predefined pressure, wherein the third predefined pressure is inferior to the second predefined pressure, and the step of acquiring (S4) the physiological signal from the first sensor (12) is performed in a time window during which the pressure in the inflatable compartment (101) is equal to the third predefined pressure, during said step of inflating (S1) and/or deflating (S2).

12. The method according to any one of claims **9** to **11,** further comprising:
- receiving the acquired physiological signal and a value of the pressure inside the inflatable compartment (101) during the acquiring (S4);
- evaluating (S6) a quality metric of the acquired physiological signal; and
- whenever the quality metric of the acquired physiological signal exceeds a predefined quality threshold, defining (S7) an optimal pressure having the value of the pressure inside the inflatable compartment (101) during the acquiring (S4), and setting (S8), as the third predefined pressure, the value of said optimal pressure;
- else, inflating (S1) the inflatable compartment (101) at a newly defined pressure, superior to the received value of pressure, and acquiring (S4) the physiological signal from the first sensor (12) while the pressure inside the inflatable compartment (101) equals said newly defined pressure.

13. The method according to claim **12,** wherein the predefined quality threshold is selected among one of the following: perfusion index, signal-to-noise ratio, motion, skewness, kurtosis, entropy, zero crossing rate, systolic wave detectors, relative power, or a combination thereof.

14. The method according to any one of claims **10** to **13,** wherein the steps of the method are periodically repeated or user-initiated.
